# EUROPEAN PATENT SPECIFICATION

(11) **EP 0 869 810 B1**
(45) Date of publication and mention of the grant of the patent: **06.10.2004**
(21) Application number: 97929227.3
(22) Date of filing: 19.06.1997
(51) Int. Cl.: A61K 38/13, A61K 47/14, A61K 47/08, A61K 9/48

(54) **CYCLOSPORIN-CONTAINING SOFT CAPSULE PREPARATIONS**
CYCLOSPORIN ENTHALTENDE WEICHKAPSELPRÄPARATE
PREPARATIONS DE GELULES MOLLES CONTENANT DE LA CYCLOSPORINE

(30) Priority: 19.06.1996 KR 9622417; 14.03.1997 KR 9708750
(43) Date of publication of application: 14.10.1998
(60) Divisional application: 03029109.0
(73) Proprietor: Novartis AG, 4056 Basel (CH); Novartis Pharma GmbH, 1230 Wien (AT)
(72) Inventor: WOO, Jong, Soo, Jangan-gu Suwon-shi Kyungki-do (KR)
(86) International application number: PCT/EP1997/003213
(87) International publication number: WO 1997/048410

(56) References cited:
- EP-A- 0 539 319
- EP-A- 0 712 631
- WO-A-91/15210
- WO-A-97/22358
- US-A- 5 589 455

## Description

The present invention relates to an, e.g. soft capsule preparation containing cyclosporin as an active ingredient. More specifically, the present invention relates to a soft capsule preparation containing a stable cyclosporin composition in a gelatin capsule shell containing a certain plasticizer and a process for preparing thereof.

Cyclosporin is a specific macromolecular (molecular weight 1202.64) cyclic peptide compound consisting of 11 amino acids, which has broad spectrum of useful pharmacological activities, particularly immuno-suppressive activity and anti-inflammatory activity. Therefore, cyclosporin has been used for suppression of inherent immunological responses of the living body, which are caused by tissue and organ transplantation, for example, transplantation of the heart, lung, liver, kidney, pancreas, bone marrow, skin and cornea, and especially the transplantation of foreign tissues and organs. In addition, cyclosporin is useful for the suppression of hematological disorders such as anemia, various autoimmune diseases such as systemic lupus erythematosus, idiopathic malabsorption syndrome, etc., and inflammatory diseases such as arthritis, rheumatoid disorders, etc. Cyclosporin is useful in treatment of protozoal diseases such as malaria, schistosomiasis, etc. , and furthermore, recently it is also used in chemotherapy.

Cyclosporin is highly lipophilic and hydrophobic. Therefore, cyclosporin is sparingly soluble in water, and as well dissolved in an organic solvent such as methanol, ethanol, acetone, ether, chloroform and the like. Due to low water-solubility of cyclosporin having above-mentioned properties, when cyclosporin is administered orally, its bioavailability is extremely low and may be greatly influenced by the conditions of each individual patient. Accordingly, it is very difficult to retain an effective therapeutic concentration. Moreover, cyclosporin may show considerable side effects such as nephrotoxicity. Thus, cyclosporin is very difficult to formulate into a preparation for oral administration due to its low water solubility. Accordingly, numerous studies have been extensively conducted to discover a preparation suitable for the effective oral administration of cyclosporin, which can provide a suitable uniform dosage and appropriate bioavailability.

In the prior art, the preparations suitable for oral administration of sparingly water-soluble cyclosporin have been usually formulated in the form of a emulsion pre-concentrate.

One typical method using this combination is taught in U.S. Patent No. 4,388,307 which issued on June 14, 1983. This patent discloses a liquid formulation of cyclosporin using ethanol. According to the method disclosed in this US Patent Specification, cyclosporin is combined with a carrier consisting of ethanol as a co-surfactant; olive oil as a vegetable oil, and a transesterification product of a natural vegetable oil triglyceride and a polyalkylene polyol as a surfactant to form the liquid formulation.

However, the resulting liquid formulation is administered as an aqueous dilution which makes it very difficult to adapt the subject to its administration and to provide a uniform dosage for oral administration.

In order to mitigate the inconvenience of diluting the cyclosporin liquid composition in water prior to oral administration, a liquid composition in the form of an emulsion pre-concentrate has been formulated into a soft capsule preparation, which is now commercially available as Sandimmune (registered trademark). In this case the cyclosporin soft capsule contains ethanol due to the solubility requirements of cyclosporin. However, since ethanol may permeate the gelatin shell of the capsule as it is volatile even at normal temperature, to prevent the volatilization of ethanol from the soft capsule preparations during storage and distribution, the soft capsule preparations may be wrapped in a special packing material, such as an aluminium-aluminum blister package.

Recently it has been possible to develop a cyclosporin preparation which has a stability during the storage period and further provides substantially no change in biological availability and its difference between individual subjects, so that the biological effect of cyclosporin can be uniformly maintained. One of the preparations developed for this purpose is disclosed in Korean Laid-open Patent Publication No. 93-113. This preparation is commercialized under the registered trademark Sandimmun Neoral. However, since this preparation also uses ethanol, it may have some disadvantages as in the prior ethanol-containing preparations, in storage stability, and changes in the ethanol content.

EP 0 539 319 A2 discloses a pharmaceutical composition comprising a cyclosorin, 1,2 propylene glycol, a mixed mono-, di- and tri-glyceride and a hydrophilic surfactant.

Accordingly, the present inventors have studied numerous combinations of various surfactants, oil components, co-surfactants etc. to find a cyclosporin composition which is stable, and provides higher bioavailability and lower difference in blood levels between individual subjects than those of the prior cyclosporin preparations in view of their pharmacokinetic properties. As a result, we have identified that a certain cyclosporin composition consisting of the components as defined below can satisfy the above-mentioned requirements, and then completed the present invention.

Therefore, it is an aspect of the present invention to provide a soft gelatin capsule preparation containing a pharmaceutical composition comprising
(i) cyclosporin as an active agent,
(ii) propylene carbonate,
(iii) a mixture of a medium chain fatty acid triglyceride and a fatty acid monoglyceride as an oil component, and
(iv) a surfactant having an HLB value from 8 to 17 which comprises a polyoxyethylene hydrogenated vegetable oil.

Even though the present invention is described herein particularly with respect to soft gelatine capsules, it is to be appreciated that the invention covers the composition itself which may be used as such e.g. as a drink solution, e.g. as Sandimmun Neoral, or be in other unit dosage forms.

In one aspect, the present invention relates to a cyclosporin-containing capsule which has a high storage stability such that there is little variation of the composition over time, and has an increased bioavailability, and which contains a composition as defined above.

To formulate such cyclosporin-containing composition into the soft capsule preparation, a gelatin shell must be used. However, when the soft capsule is formulated with the general capsule shell containing glycerine as plasticizer, the soft capsule preparation has some disadvantages in that the emulsified state of the emulsion pre-concentrate may be changed due to the inflow of glycerine into the emulsion and, therefore, the solubility of cyclosporin is significantly lower to result in the precipitation of cyclosporin from the emulsion.

Accordingly, in the present invention preferably a gelatin shell using a mixture of propylene glycol and polyethylene glycol, not glycerine, as a plasticizer is selected for the soft capsule shell, which can solve the problem related with inflow of glycerine.

However, when the capsule shell band containing propylene glycol and polyethylene glycol according to the present invention is prepared by means of a water-cooling method which is conventionally used for a cooling drum, it is not easily removed from the drum. Such removability of the capsule shell band from the cooling drum may be improved by over-cooling the cooling drum by continuously circulating a cooling water to reduce temperature of the band to about 17°C. However, the capsule shell band which is cooled to lower temperature may provide a low extent of seal during the encapsulation process and may cause lowering of productivity.

Therefore, the process for preparing the gelatin shell band not containing glycerine plasticizer according to the present invention adopts an air-cooling method, instead of the prior water-cooling method, in which the capsule shell band can be cooled down to the optimum temperature by providing an air flow from a fan and therefore, can be readily removed from the cooling drum and further, it is maintained at the optimum temperature of about 21°C to increase the extent of seal in the encapsulation process and ensure a high productivity.

As mentioned above, the present products can be produced by using the glycerine-free gelatin capsule shell and applying the air-cooling method to the composition which does not contain ethanol as a low boiling volatile solvent and therefore, has a high storage stability such that there is little variation of the composition over time, and has an increase bioavailability.

More specifically, the present invention relates to a cyclosporin preparation, which comprises a composition containing
1) cyclosporin as an active ingredient;
2) propylene carbonate;
3) a mixture of a medium chain fatty acid triglyceride and fatty acid monoglyceride as an oil component and
4) a surfactant having an HLB (Hydrophilic Lipophilic Balance) value of 8 to 17, which comprises a polyoxyethylene hydrogenated vegetable oil, in a gelatin shell containing polyethylene glycol and propylene glycol as a plasticizer.

Such a composition, which is also a composition of the invention, may optionally additionally comprise any other component as described herein, if desired in the amounts described herein.

Cyclosporin, which is used as the pharmaceutically active ingredient in the composition according to the present invention, is a cyclic peptide compound having useful immuno-suppressive activity and anti-inflammatory activity as described above. Although cyclosporin A, B, C, D, G and the like can be used as the cyclosporin component in the present invention. Cyclosporin A is mostly preferred since its clinical effectiveness and pharmacological properties are well established in the art.

As a further component in the composition according to the present invention, and which may act as a co-surfactant polyethylene glycol which has a high boiling point, is non-volatile, does not permeate the gelatin shell of the soft capsule, and has a high solubility for cyclosporin, may be used as a hydrophilic substance. In the composition according to the present invention, although any polyethylene glycol which can be liquified can be used, polyethylene glycol (PEG) having molecular weight of 200 to 600, particularly PEG 200, can be preferably used.

Propylene carbonate (b.p. about 242°C) is used as a non-hydrophilic component. In the present invention, the mixture of non-hydrophilic substance and hydrophilic substance as defined above can also be used. When a mixture of polyethylene glycol and propylene carbonate is used as the component in the present invention, they can be generally combined in the ratio of 1:0.1-5, preferably 1:0.1-3, most preferably 1:0.2-2, on the basis of weight.

In the present invention, the use of polyethylene glycol and propylene carbonate provides certain advantages. That is, the stability of the cyclosporin-containing composition during storage is improved and therefore the contents of the components contained therein are substantially uniformly maintained.

Furthermore, the use of propylene carbonate can even increase the solubility of the active ingredient cyclosporin and inhibit the inflow of water from the gelatin capsule shell into the composition to provide a more stable composition.

In the composition of the present invention, the second component is used preferably in the ratio of 0.1 to 10 parts by weight, more preferably 0.5 to 8 parts by weight, and most preferably 1 to 5 parts by weight, per 1 part by weight of cyclosporin.

The third component used in the emulsion pre-concentrate according to the present invention is an oil component. As the oil component in the present invention, a mixture of a medium chain fatty acid triglyceride and fatty acid monoglyceride is used. The esterified compound of fatty acid and primary alcohol which can be used in the present invention may include an esterified compound of fatty acid having 8 to 20 carbon atoms and primary alcohol having 2 to 3 carbon atoms, for example, isopropyl myristate, isopropyl palmitate, ethyl linoleate, ethyl oleate, etc., with an esterified compound of linoleic acid and ethanol being particularly preferably. In addition, as the medium chain fatty acid triglyceride a triglyceride of saturated fatty acid having 8 to 10 carbon atoms can be used with caprylic /capric acid triglyceride as a vegetable oil triglyceride of saturated fatty acid being most preferably used. The fatty acid monoglyceride which is used in the oil component in the present invention includes a monoglyceride of fatty acid having 18 to 20 carbon atoms, particularly monoglyceride of oleic acid.

In a microemulsion pre-concentrate according to the present invention, the oil component may be used in the ratio of 1 to 10 parts by weight, preferably 2 to 6 parts by weight, per 1 part by weight of cyclosporin.

Preferably fatty acid monoglyceride and fatty acid ester are present as oil component, e.g. in the ratio 1:1 to 1:2, e.g. 1:1 to 1:1.2.

Optionally caprylic/capric acid triglyceride is also present e.g. in a ratio to ethyl linoleate of from 1:0.1 to 0.2.

In the oil mixture used as the oil component according to the present invention, the mixing ratio of fatty acid monoglyceride: an esterified compound of fatty acid and primary alcohol: medium chain fatty acid triglyceride may be generally in the range of 1:0.1-5; 0.1-10 preferably in the range of 1:0.1-3.0:0.1-3.0, on the basis of weight.

The fourth component used in the composition according to the present invention is a surfactant. The suitable surfactants for use in the present invention include any of pharmaceutically acceptable surfactants having an HLB (Hydrophilic Lipophilic Balance) value of 8 to 17, which are capable of stably emulsifying the lipophilic portion of the composition comprising the cyclosporin-containing oil component and the hydrophilic portion comprising the co-surfactant in water to form a stable microemulsion, and which are polyoxyethylene products of hydrogenated vegetable oils, optionally further including polyoxyethylene-sorbitan-fatty acid esters, and the like, for example, NIKKOL HCO-50, NIKKOL HCO_40, NIKKOL HCO-60, TWEEN 20, TWEEN 21, TWEEN 40, TWEEN 60, TWEEN 80, TWEEN 81, etc. Particularly, a polyoxyethylene (50) hydrogenated castor oil which is commercialised under the trade mark NIKKOL HCO-50 (NIKKO Chemical Co., Ltd.) and a polyoxyethlyene (20) sorbitan monolaurate which is commercialised under the trade mark TWEEN 20 (ICI Chemicals), having an acid value below 1, a saponification value of about 48-56, a hydroxyl value of about 45-55 and pH value (5%) of 4.5-7.0, can be preferably used.

The surfactant can include any one of the above-mentioned surfactants alone or, preferably, in a combination of two or more surfactants selected from the above surfactants. In the composition according to the present invention, the surfactants can be used in the ratio of 1 to 10 parts by weight, preferably in the ratio of 2 to 8 parts by weight, per 1 part by weight of cyclosporin.

In addition, when the mixture of two surfactants, i.e. polyoxyethylene (50) hydrogenated castor oil and polyoxyethylene (20) sorbitan monolaurate is used in the composition of the present invention, the constitutional ratio of polyoxyethylene (50) hydrogenated castor oil; polyoxyethylene (20) sorbitan monolaurate is preferably in the range of 1:0.1-5, more preferably in the range of 1:0.5-4, on the basis of weight.

In the composition according to the present invention, the four components are present preferably in the ratio of cyclosporin: second component; oil component; surfactant = 1 : 0.1- 10 : 1-10 : 1-10, and more preferably in the ratio of cyclosporin: second component: oil component : surfactant = 1 : 0.5-8: 2-6 : 2-8, by weight.

In addition to this composition, the composition illustrated in the following examples can be mentioned as further preferred compositions according to the present invention.

For oral administration, the composition of the present invention containing the above-mentioned components can be formulated into the form of a soft capsule.

Since the soft capsule preparation according to the present invention does not use ethanol as the low-boiling volatile solvent, it is pharmaceutically stable and can establish the desired improvements including improvement of bioavailability.

However, it may be difficult to reproductively prepare as a conventional soft capsule shell by means of a conventional method for preparation of soft capsules. When the soft capsule is formulated with the conventional capsule shell containing glycerine as plasticizer, the soft capsule thus prepared may have some disadvantages in that the emulsified state of the emulsion pre-concentrate may be changed due to the inflow of glycerine into the emulsion and therefore, the solubility of cyclosporin is significantly lowered which may result in the precipitation of cyclosporin from the emulsion.

Accordingly, in another aspect the present invention it is found that when the capsule shell is formulated by using a mixture of polyethylene glycol and propylene glycol, not glycerine, as a plasticizer, the soft capsule preparation which is stable for a long period can be obtained. Although any polyethylene glycol which can be liquified may be used as a plasticizer, it is preferable to use polyethylene glycol having molecular weight of 200 to 600.

Particularly, polyethylene glycol 200 is preferably used. In the soft capsule shell according to the present invention, the mixture of polyethyene glycol and propylene glycol is preferably used in the ratio of 0.1 to 0.5 parts by weight, more preferably 0.1 to 0.4 parts by weight and most preferably 0.2 to 0.3 parts by weight, with respect to one part by weight of gelatin used for preparing the capsule shell. In the mixture of polyethylene glycol and propylene glycol as the plasticizer, propylene glycol is combined preferably in the ratio of 1 to 10 parts by weight, more preferably 3 to 8 parts by weight and most preferably 3 to 6 parts by weight, with respect to one part by weight of polyethylene glycol.

In order to increase the removability of the soft capsule shell band from the cooling drum, the process for preparing the gelatin capsule shell band according to the present invention adopts the air-cooling method, instead of the water-cooling method. According to this air-cooling method, since the capsule shell band is not overheated and can be readily removed from the cooling drum while maintaining the optimum temperature of about 21°C, the extent of seal in the encapsulation process is high to ensure a high productivity and therefore, the process can be efficiently conducted.

In preparing the soft capsule according to the present invention, a suitable air volume flow for the cooling drum to cool the capsule shell is preferably 5 to 15m³/min., most preferably about 10m³/min.

In formulating the composition according to the present invention into soft capsules, the capsule preparation can further contain, if necessary, pharmaceutically acceptable additives which are conventionally utilized in the preparation of soft capsules. Such additives include, for example, lecithin, viscosity regulator, perfume (e.g. peppermint oil, etc.), anti-oxidant (e.g. tocopherol, Vitamin E etc.), preservative (e.g. parabene, etc.), coloring agent, amino acids, etc.

The soft capsule preparation according to the present invention can be prepared by uniformly mixing the co-surfactant, the oil component and the surfactant, dissolving cyclosporin therein while stirring and gently warming to the temperature of approximately 60°C, and then formulating the resulting concentrate, with or without the above-mentioned pharmaceutically acceptable additives conventionally utilized in preparation of soft capsules, with the gelatin shell containing polyethylene glycol and propylene glycol as the plasticizer in a machine for preparing soft capsules by means of the air-cooled method into the desired suitable cyclosporin soft capsule.

The compositions and preparations of the present invention are useful for the same indications and may be administered in the same way and dosage range as known cyclosporin compositions, if necessary adjusting the dose on the basis of standard bioavailability trials in animals, e.g. dogs, or humans, e.g. as described hereinafter.

Insofar as details of the compositions of any excipients or components are not specifically described herein, these are described in the literature, e.g. H.P. Fiedler, Lexikon der Hilfsstoffe, Edito Cantor Verlag, Aulendorf, Germany, 4th Edition 1996, Handbook of Pharmaceutical Excipients, American Pharmaceutical Association, Washington, and The Pharmaceutical Society, London, 2nd Edition, 1994 and Korean Patent Application 94-29208 filed 9.11.94.

The present invention will be more specifically illustrated by the following examples.

### EXAMPLE 1:

| Component | Content (mg/Cap.) |
|---|---|
| Cyclosporin | 25 |
| polyethylene glycol 200 | 45 |
| propylene carbonate | 25 |
| polyoxyethylene (50) hydrogenated castor oil | 35 |
| polyoxyethylene (20) sorbitan monolaurate | 85 |
| ethyl linoleate | 40 |
| caprylic/capric acid triglyceride | 5 |
| oleic acid monoglyceride | 35 |
| total | 295 mg |

### Reference EXAMPLE 2:

| Component | Content /mg/Cap.) |
|---|---|
| Cyclosporin | 25 |
| polyethylene glycol 200 | 70 |
| polyoxyethylene (50) hydrogenated castor oil | 35 |
| polyoxyethylene (20) sorbitan monolaurate | 85 |
| ethyl linoleate | 40 |
| caprylic /capric acid triglyceride | 5 |
| oleic acid monoglyceride | 35 |
| total | 295 mg |

### EXAMPLE 3:

| Component | Content (mg/Cap.) |
|---|---|
| Cyclosporin | 25 |
| polyethylene glycol 200 | 100 |
| propylene carbonate | 50 |
| polyoxyethylene (50) hydrogenated castor oil | 35 |
| polyoxyethylene (20) sorbitan monolaurate | 85 |
| ethyl linoleate | 40 |
| caprylic /capric acid triglyceride | 5 |
| oleic acid monoglyceride | 35 |
| total | 375 mg |

### EXAMPLE 4:

| Component | Content (mg/Cap.) |
|---|---|
| Cyclosporin | 25 |
| polyethylene glycol 200 | 45 |
| propylene carbonate | 25 |
| polyoxyethylene (50) hydrogenated castor oil | 50 |
| polyoxyethylene (20) sorbitan monolaurate | 100 |
| ethyl linoleate | 40 |
| caprylic /capric acid triglyceride | 5 |
| oleic acid monoglyceride | 35 |
| total | 325 mg |

### EXAMPLE 5:

| Component | Content (mg/Cap.) |
|---|---|
| Cyclosporin | 25 |
| polyethylene glycol 200 | 45 |
| propylene carbonate | 25 |
| polyoxyethylene (50) hydrogenated castor oil | 35 |
| polyoxyethylene (20) sorbitan monolaurate | 85 |
| ethyl linoleate | 80 |
| caprylic /capric acid triglyceride | 10 |
| oleic acid monoglyceride | 70 |
| total | 375 mg |

### EXAMPLE 6:

The soft capsule preparation was prepared from the composition of Example 1 using the following composition for capsule shell and then the change in the property and condition of the content due to the inflow of glycerine was visually observed.

| 6.1 | (control group) | |
|---|---|---|
| | Component | Weight ratio |
| | gelatin | 20 |
| | purified water | 16 |
| | glycerine | 9 |

| 6.2 | (test group) | |
|---|---|---|
| | Component | Weight ratio |
| | gelatin | 20 |
| | purified water | 16 |
| | propylene glycol | 4 |
| | polyethylene glycol 200 | 1 |

The results as observed are described in the following Table 1.

**Table 1:**

| Stability of the content of the capsule preparation according to the capsule shell | | | | | | |
|---|---|---|---|---|---|---|
| Composition of Capsule | Just after formulation | After 1 day | After 2 days | After 5 days | After 10 days | After 30 days |
| Control group | 0 | + | + | ++ | +++ | +++ |
| Test group | 0 | 0 | 0 | 0 | 0 | 0 |

| | | | | | | |
|---|---|---|---|---|---|---|
| Note: 0 = the content is stable | | | | | | |
| + = poor emulsification | | | | | | |
| ++ = slight precipitation | | | | | | |
| +++ = precipitation | | | | | | |

As can be seen from the result described in table 1, the capsule preparation prepared using the composition of 6.1 control group containing glycerine as the plasticizer causes some problems including the formation of precipitation due to the inflow of glycerine, whereas the capsule preparation prepared using the composition of 6.2 test group containing polyethylene glycol and propylene glycol as the plasticizer maintains the stable condition.

### EXAMPLE 7:

The soft capsule preparation having the composition of Example 1 was prepared using the composition of the capsule shell of 6.2 test group used in the above Example 6 by means of the water-cooling method (water temperature about 12°C) and the air-cooling method (air volume flow about 10 m³/min), respectively.

In each case, the removability of the capsule shell band from the cooling drum was observed and compared. The result as observed is described in the following Table 2.

**Table 2:**

| Removability of the gelatin shell band from the cooling drum depending on the cooling method | | |
|---|---|---|
| (Unit: degree of angle) | | |
| Shell Composition | Water-cooling Method | Air-cooling Method |
| Example 6.2 | > 100 degree (poor removability) | < 50 degree (good removability) |

As can be seen from the result described in the Table 2 above, the soft capsule preparation prepared by the air-cooling method according to the present invention shows a far better removability from the cooling drum in comparisoin with that prepared by the water-cooling method. Specifically, it is generally considered that if the degree of angle for removing the gelatin shell band from the cooling drum is about 70 or more, the removability is poor, and if the degree of angle for removing the shell band is below about 70, the removability is good. The soft capsule preparation prepared by the water-cooling method is not satisfactorily removed from the cooling drum even when the preparation is removed at the angle of 100 degrees or more.

Contrary to this, the soft capsule preparation prepared by the air-cooling ethod according to the present invention can be easily removed from the cooling drum at the angle of 50 degree and below and therefore, can provide a good sealing strength and productivity.

### EXAMPLE 8:

The bioavailability of the preparation prepared by encapsulating the composition of Example 1 with the gelatin shell having the composition of Example 6.2 as the test preparation was compared with the bioavailability of the commercial product containing ethanol, SANDIMMUN Capsule, as the control preparation to estimate the influence of the cyclosporin preparation according to the present invention on the bioavailability of cyclosporin and its difference between respective subjects.

In this experiment, both of the test preparation and the control preparation were administered in an amount of 300 mg as cyclosporin per kg of rabbit.

Rabbits were uniformly fed with the conventional rabbit solid feed composition for 4 days or more under the same condition in wire cages. When the oral preparation were administered, rabbits were fasted for 48 hours in a restraint cage made of steel, during which rabbits were allowed to freely take water.

Levin's tube having a diameter of 5 mm was interposed by the depth of 30 cm through the esophagus after the surface of the Levin's tube was coated with vaseline in order to reduce friction. Each of the test preparations and the control preparation was emulsified with 50 ml of water and then introduced into a syringe which is attached to the Levin's tube. Ear veins of rabbit were dilated using xylene and then blood was taken from each rabbit's ear vein before the test and after 0.5, 1, 1.5, 2, 3, 4, 6, 10 and 24 hours by means of heparin-treated disposable syringe. To 1 ml of blood thus obtained were added 0.5 ml of aqueous saturated sodium chloride solution and 2 ml of ether, and then the mixture was shaken for 5 minutes and centrifuged with 5000 rpm for 10 minutes to separate the supernatant (ether layer). 1 ml of the supernatant was collected and then developed in an activated silica sep-pak^{R} (Waters). The developed sep-pak was washed with 5 ml of n-hexane and eluated with 2 ml of methanol. The eluate was evaporated to dryness in nitrogen gas under reduced pressure. The residue was analysed by means of HPLC (High Performance Liquid Chromatography) [HPLC condition: column µ-Bondapak^{R} C₁₈ (Waters), mobile phase CH₃CN: MeOH; H₂O - 55: 15 : 30, detection 210 nm, flow rate 1.0 ml /min., column temperature 70°C, sensitivity 0.01 Aufs. injection volume 100 µl].

The results obtained from the test preparation and the control preparation are illustrated in the following Table 3:

**Table 3:**

| Bioavailability of the test preparation of the present invention and the commercial product (SANDIMMUN^{R}) | | | | | |
|---|---|---|---|---|---|
| Parameter | Control | Prep. (A) | Test Prep. (B) | | P (B/A) |
| | M ± S.D. (n=6) | CV % (S.D./M) | M ± S.D. (n=6) | CV % (S.D./M) | |
| AUC (µg.hr/ml) | 13.5±10.0 | 74.0 % | 57.0±17.0 | 29.8 % | 4.1 |
| Cₘₐₓ (µg.hr/ml) | 0.8±0.3 | 37.5 % | 6.0±1.5 | 25.0 % | 7.5 |
| Note: - AUC =: Area under the blood concentration curve - Cₘₐₓ =: Maximum blood concentration of cyclosporin - M± S.D.=: Mean value ± Standard deviation - CV =: Ratio of standard deviation to mean value - P(B/A) =: Ratio of mean value of the test preparation to mean value of the control preparation | | | | | |

As can be seen from the above table, the test preparation shows the increased AUC and Cₘₐₓ values which are about 4 times or more and about 7 times or more, respectively, as high as those of the control preparation. Accordingly, it can be identified that the bioavailability of the test preparation is significantly increased in comparison with that of the control preparation. In addition, the test preparation of the present invention exhibits an effect of decreasing the difference between respective test subjects (CV %) by about 2 times or more in AUC value and by about 1.5 times in Cₘₐₓ value, in comparison with the control preparation.

Accordingly, it could be determined that when the soft capsule preparation according to the present invention is administered per oral, it shows the increased bioavailability of cyclosporin about 4 times as high as that of the prior commercial product containing ethanol, SANDIMMUN^{R} Capsule and also a decrease of the difference between cyclosporin bioavailabilities in respective subjects, and at the same time, remains stable without any change during the long term storage. Thus, it is apparent that the soft capsule preparation according to the present invention provides a significant improvement in the field of preparation of cyclosporin soft capsules.

### EXAMPLE 9:

Soft gels are made up containing:

| | I | II |
|---|---|---|
| Cyclosporin A | 25 mg | 100 mg |
| Polyethylene glycol 200 | 45 mg | 180 mg |
| Propylene carbonate | 25 mg | 100 mg |
| Polyoxyethylene 50-hydrogenated | | |
| castor oil | 40 mg | 160 mg |
| Polysorbate 20 | 85 mg | 340 mg |
| Ethyl linoleate | 40 mg | 160 mg |
| Glyceryl mono-oleate | 40 mg | 160 mg |
| Vitamin E | 1 mg | 4 mg |
| Total | 301 mg | 1204 mg |

## Claims

1. A soft gelatin capsule preparation containing a pharmaceutical composition comprising
(i) cyclosporin as an active agent,
(ii) propylene carbonate,
(iii) a mixture of a medium chain fatty acid triglyceride and a fatty acid monoglyceride as an oil component, and
(iv) a surfactant having an HLB value from 8 to 17 which comprises a polyoxyethylene hydrogenated vegetable oil.

2. A composition according to claim 1 wherein said cyclosporin is cyclosporin A.

3. A composition according to any preceding claim wherein the ratio of the component (ii) : cyclosporin is 1-5 : 1, on the basis of weight.

4. A composition according to any preceding claim wherein the medium chain fatty acid triglyceride comprises caprylic/capric acid triglyceride.

5. A composition according to any preceding claim wherein the fatty acid monoglyceride is a monoglyceride of oleic acid.

6. A composition according to any preceding claim wherein an esterified compound of fatty acid and primary alcohol is additionally present.

7. A composition according to claim 6 wherein the esterified compound of fatty acid and primary alcohol is ethyl linoleate.

8. A composition according to any preceding claim wherein the ratio of the oil component: cyclosporin is 2-6 : 1, on the basis of weight.

9. A composition according to claim 6, 7 or 8 wherein the ratio of fatty acid monoglyceride : esterified compound of fatty acid and primary alcohol : medium chain fatty acid triglyceride is 1:0.1-3.0: 0.1-3.0, on the basis of weight.

10. A composition according to any preceding claim wherein a mixture of a polyoxyethylene hydrogenated vegetable oil and polyoxethylene sorbitan fatty acid ester is present.

11. A composition according to any preceding claim wherein the ratio of the surfactant: cyclosporin is 2-8 : 1, on the basis of weight.

12. A composition according to claim 10 or 11 wherein said surfactant is a mixed surfactant consisting of polyoxyethylene(50) hydrogenated castor oil : polyoxyethylene(20) sorbitan monolaurate in the ratio of 1 : 0.5-4, on the basis of weight.

13. A composition according to any preceding claim wherein the ratio of cyclosporin : component (ii) : oil component: surfactant is 1:0.5-8 : 2-6 : 2-8, on the basis of weight.

14. A composition according to any preceding claim which is ethanol-free.

15. A composition according to any preceding claim wherein the soft gelatine capsule contains polyethylene glycol and propylene glycol as a plasticizer.

16. A composition according to claim 15 wherein the ratio of polyethylene glycol and propylene glycol : gelatin is 0.2-0.3 : 1, on the basis of weight.

17. A composition according to claim 15 or 16 wherein the ratio of the plasticizer polyethylene glycol : propylene glycol is 1 : 3-6, on the basis of weight.

## Patentansprüche

1. Weichgelatinekapselzubereitung, die eine pharmazeutische Zusammensetzung enthält, welche umfasst
(i) Cyclosporin als Wirkstoff,
(ii) Propylencarbonat,
(iii) ein Gemisch aus einem mittelkettigen Fettsäuretriglycerid und einem Fettsäuremonoglycerid als Ölkomponente und
(iv) ein Tensid mit einem HLB-Wert von 8 bis 17, das ein Polyoxyethylen-hydriertes Pflanzenöl umfasst.

2. Zusammensetzung nach Anspruch 1, worin das Cyclosporin Cyclosporin A ist.

3. Zusammensetzung nach einem der vorhergehenden Ansprüche, worin das Verhältnis von Komponente (ii) : Cyclosporin auf Gewichtsbasis 1 bis 5 : 1 beträgt.

4. Zusammensetzung nach einem der vorhergehenden Ansprüche, worin das mittelkettige Fettsäuretriglycerid Caprylsäure-Caprinsäuretriglycerid umfasst.

5. Zusammensetzung nach einem der vorhergehenden Ansprüche, worin das Fettsäuremonoglycerid ein Monoglycerid von Ölsäure ist.

6. Zusammensetzung nach einem der vorhergehenden Ansprüche, worin zusätzlich eine veresterte Verbindung aus einer Fettsäure und einem primären Alkohol vorhanden ist.

7. Zusammensetzung nach Anspruch 6, worin die veresterte Verbindung aus einer Fettsäure und einem primären Alkohol Ethyllinoleat ist.

8. Zusammensetzung nach einem der vorhergehenden Ansprüche, worin das Verhältnis von Ölkomponente ; Cyclosporin auf Gewichtsbasis 2 bis 6 : 1 beträgt.

9. Zusammensetzung nach Anspruch 6, 7 oder 8, worin das Verhältnis von Fettsäuremonoglycerid : veresterter Verbindung aus der Fettsäure und dem primären Alkohol; mittelkettigem Fettsäuretriglycerid auf Gewichtsbasis 1 : 0,1 bis 3,0 : 0,1 bis 3,0 beträgt.

10. Zusammensetzung nach einem der vorhergehenden Ansprüche, worin ein Gemisch aus einem Polyoxyethylen-hydriertem Pflanzenöl und einem Polyoxyethylen-Sorbitanfettsäureester vorhanden ist.

11. Zusammensetzung nach einem der vorhergehenden Ansprüche, worin das Verhältnis von Tensid: Cyclosporin auf Gewichtsbasis 2 bis 8 : 1 beträgt.

12. Zusammensetzung nach Anspruch 10 oder 11, worin das Tensid ein Tensidgemisch ist, das aus Polyoxyethylen(50)-hydriertem Ricinusöl : Polyoxyethylen(20)-Sorbitanmonolaurat in einem Gewichtsverhältnis von 1 : 0,5 bis 4 besteht.

13. Zusammensetzung nach einem der vorhergehenden Ansprüche, worin das Verhältnis aus Cyclosporin : Komponente (ii) : Ölkomponente : Tensid auf Gewichtsbasis 1 : 05, bis 8 : 2 bis 6 : 2 bis 8 beträgt.

14. Zusammensetzung nach einem der vorhergehenden Ansprüche, die frei an Ethanol ist.

15. Zusammensetzung nach einem der vorhergehenden Ansprüche, worin die Weichgelatinekapsel Polyethylenglycol und Propylenglycol als einen Weichmacher enthält.

16. Zusammensetzung nach Anspruch 15, worin das Verhältnis von Polyethylenglycol und Propylenglycol : Gelatine auf Gewichtsbasis 0,2 bis 0,3 : 1 beträgt.

17. Zusammensetzung nach Anspruch 15 oder 16, worin das Verhältnis des Weichmachers Polyethylenglycol : Propylenglycol auf Gewichtsbasis 1 : 3 bis 6 beträgt.

## Revendications

1. Préparation de capsule de gélatine molle contenant une composition pharmaceutique comprenant
(i) de la cyclosporine en tant qu'agent actif,
(ii) du carbonate de propylène,
(iii) un mélange d'un triglycéride d'acide gras à chaîne moyenne et d'un monoglycéride d'acide gras comme composant huileux, et
(iv) un tensioactif ayant une valeur de HLB de 8 à 17 qui comprend une huile végétale hydrogénée polyoxyéthylénée.

2. Composition selon la revendication 1, dans laquelle ladite cyclosporine est la cyclosporine A.

3. Composition selon l'une quelconque des revendications précédentes, dans laquelle le rapport du composant (ii) à la cyclosporine est de 1-5 : 1, sur la base du poids.

4. Composition selon l'une quelconque des revendications précédentes, dans laquelle le triglycéride d'acide gras à chaîne moyenne comprend un triglycéride d'acide caprylique/caprique.

5. Composition selon l'une quelconque des revendications précédentes, dans laquelle le monoglycéride d'acide gras est un monoglycéride d'acide oléique.

6. Composition selon l'une quelconque des revendications précédentes, dans laquelle un composé estérifié d'acide gras et d'alcool primaire est en outre présent.

7. Composition selon la revendication 6, dans laquelle le composé estérifié d'acide gras et d'alcool primaire est le linoléate d'éthyle.

8. Composition selon l'une quelconque des revendications précédentes, dans laquelle le rapport du composant huileux à la cyclosporine est de 2-6 : 1, sur la base du poids.

9. Composition selon la revendication 6, 7 ou 8, dans laquelle le rapport monoglycéride d'acide gras : composé estérifié d'acide gras et d'alcool primaire : triglycéride d'acide gras à chaîne moyenne est de 1 : 0,1-3,0 : 0,1-3,0, sur la base du poids.

10. Composition selon l'une quelconque des revendications précédentes, dans laquelle un mélange d'une huile végétale hydrogénée polyoxyéthylénée et d'ester d'acide gras et de sorbitane polyoxyéthyléné est présent.

11. Composition selon l'une quelconque des revendications précédentes, dans laquelle le rapport du tensioactif à la cyclosporine est de 2-8 : 1, sur la base du poids.

12. Composition selon la revendication 10 ou 11, dans laquelle ledit tensioactif est un tensioactif mixte constitué d'huile de ricin hydrogénée polyoxyéthylénée(50) et de monolaurate de sorbitane polyoxyéthyléné(20) dans un rapport de 1 : 0,5-4, sur la base du poids.

13. Composition selon l'une quelconque des revendications précédentes, dans laquelle le rapport cyclosporine : composant (ii) : composant huileux : tensioactif est de 1 : 0,5-8 : 2-6 : 2-8, sur la base du poids.

14. Composition selon l'une quelconque des revendications précédentes, qui est exempte d'éthanol.

15. Composition selon l'une quelconque des revendications précédentes, dans laquelle la capsule de gélatine molle contient du polyéthylèneglycol et du propylèneglycol en tant que plastifiant.

16. Composition selon la revendication 15, dans laquelle le rapport du polyéthylèneglycol et du propylèneglycol à la gélatine est de 0,2-0,3 : 1, sur la base du poids.

17. Composition selon la revendication 15 ou 16, dans laquelle le rapport du plastifiant polyéthylèneglycol : propylèneglycol est de 1 : 3-6, sur la base du poids.
